# EUROPEAN PATENT SPECIFICATION

(11) **EP 1 161 268 B1**
(45) Date of publication and mention of the grant of the patent: **31.08.2005**
(21) Application number: 00909499.6
(22) Date of filing: 13.03.2000
(51) Int. Cl.: A61L 27/00, A61L 31/10, A61L 33/06, A61F 2/06

(54) **BIOCOMPATIBLE ENDOPROSTHESES**
BIOVERTRÄGLICHE ENDOPROTHESEN
ENDOPROTHESES BIOCOMPATIBLES

(30) Priority: 13.03.1999 GB 9905759
(43) Date of publication of application: 12.12.2001
(73) Proprietor: Biointeractions Ltd, Reading RG6 2EF (GB)
(72) Inventor: LUTHRA, Ajay, Kumar, Middlesex HA2 8UA (GB); SANDHU, Shivpal, Singh, Berkshire SL3 7PW (GB); SHARMA, Anoop, Kurmar, Middlesex TW5 9RG (GB); HUDSON, John, Overton, Leicester LE3 8AG (GB)
(74) Representative: Wolff, Francis Paul
(86) International application number: PCT/GB2000/000913
(87) International publication number: WO 2000/054822

(56) References cited:
- EP-A- 0 797 963
- WO-A-97/41164
- DE-A- 4 407 079
- DE-A- 19 524 653
- US-A- 5 624 508
- US-A- 5 772 668

## Description

This invention relates to expandable endoprosthetic devices used to improve the patency of various vessels in the human body. Such endoprostheses are commonly known as "stent grafts".

The development of minimally invasive technology has enabled vascular disease to be treated without the need for surgery. Mechanical tools and devices can now be delivered to the site of a vascular lesion via the vascular canals themselves using the so-called "Percutaneous Transluminal" methods.

Arteries may be stenosed or occluded by arteriosclerosis or atherosclerosis, in which the arteries become hardened and partially or fully blocked by build up of lipid material. Such blockage reduces or stops blood flow, and can cause damage to the limbs or organs distal to the stenosis.

Removal of plaque by atherectomy, or pushing the plaque aside by balloon angioplasty (PTA), are two of the techniques now available to interventionists using minimally invasive technology. Vascular atherectomy and PTA, (percutaneous transluminal angioplasty), can recanalise a vessel and restore blood flow, but the result may not be permanent, or there may be complications.

Complications include the following: -
1. Elastic recoil, where the tissue partially returns to its original configuration after angioplasty,
2. Tissue re-growth.
3. Intimal flap. A flap of tissue partially attached to the arterial wall.

The simplest device used to prevent these complications is the insertion of a stent. A stent is a cylindrical device, which is placed inside a vessel and presses against the wall. Such stents must be capable of being folded or compacted in order to be delivered through the vasculature, and then expanded to a substantially cylindrical form at the site of the lesion.

Many different types of stent are available, but they fall into two categories: a) balloon expandable, and b) self expandable. The former is made of a malleable material and is mounted on a balloon catheter and expanded at the site of the lesion. The latter are elastic devices, which are compacted and restrained during deployment, and then released at the site.

A special type of self-expanding stent is made from Nitinol, which is a so-called "shape memory alloy", i.e. an alloy which can be pre-formed into a first preferred shape while hot, cooled down and folded into a second shape, the second shape remaining fixed until the alloy is heated whereupon it returns to its first preferred shape.

Use of such alloys advantageously permits the stent to be folded from a first preferred shape into a second shape which is small enough to fit into a catheter. After delivery to the site of the lesion, the stent returns to its first preferred shape due to the heat of the patient's body.

Nitinol may also have the property of "superelasticity", after reaching normal body temperature.

A superelastic material is a material which has elastic properties such that a large change in strain is accompanied by a relatively small change in stress. That is, a material which has a modulus of elasticity (Young's modulus), close to zero.

However, stents on their own cannot prevent all complications. While they can prevent elastic recoil, and control an intimal flap, they cannot prevent continued tissue growth leading to re-stenosis. Connective tissue can grow through the spaces in the stent.

To deal with this problem, a new generation of endoprostheses was developed where the stent was covered by a continuous tubular element. This continuous tubular element is usually a woven or knitted textile fabric, placed on the outside of the stent. Alternately, the continuous tubular element may be expanded polytetrafluoroethylene (PTFE). The continuous tubular element is therefore trapped between the stent and the arterial wall. Such a continuous tubular element is designed to form a barrier to prevent tissue re-growth.

It is normal to describe an uncovered or bare stent as a "stent", whereas a covered stent or "stent graft" is referred to as an endoprosthesis. However, it is common for both devices to be referred to as Endoprostheses.

The covered stent has another important use in the area of vascular aneurisms. Aneurisms are caused by a weakening in the wall of an artery, which allows the wall to dilate and swell out into a balloon like shape. This weakening gives the danger of rupture, which can have very serious consequences including death.

Endoprostheses may be used to bridge the aneurism inside the artery and so make an "internal bypass" of the aneurism.

The problem is that the materials used for both the stent and the covering are all thrombogenic to some degree. They therefore create a reaction with the blood to cause clotting. In order to control this clotting, the covering material must be made permeable. This permeability allows the new tissue growth to be through the pores of the material, and adhere strongly to it, thus preventing a clot becoming mobile and possibly cause an occlusion downstream.

This permeability coupled with the slight thombogenicity of the materials, means that the covering does not highly prevent tissue re-growth, and the possibility of re-stenosis.

The present invention relates to a flexible endoprosthesis as defined in claim 1 used to treat stenosis, which occurs in diseased arteries. The invention has a special function in reducing or preventing restenosis after interventional treatment. The endoprosthesis comprises a stent device coated with or encapsulated in a biocompatible polymer layer.

The configuration of the device consists of an array of cylindrical elements arranged internally or externally and secured to a continuous tubular element. The cylindrical elements may also be sandwiched between two continuous tubular elements. These composite devices are described as Arrayed Stent Grafts.

The shape of the cylindrical element is one where a suitable wire is wound once around a mandrel in a zigzag fashion and henceforth described as a crown element, or in general as a stent element.

The said continuous tubular element functions to connect the stent elements, to act as a barrier for tissue growth and for the attachment of biocompatible materials. The construction of the continuous tubular element ranges from woven or knitted fabric to a number of tubular extrusions.

The arrayed stent graft is either coated by, or encapsulated into, a highly biocompatible material in order to isolate those less biocompatible materials, used in the construction, from the blood and other body fluids or tissue.

A diseased artery may become stenosed. The mechanism of stenosis and its recurrence, restenosis, is not fully understood. There is general consensus that restenosis is a series of complex cascading events, which involve inflammatory and thrombotic mechanisms, intimal hyperplasia and vessel recoil. The biocompatible material serves to reduce the series of complex cascading events of an artificial implant such as the arrayed stent graft, thus ensuring that the device is highly biocompatible.

The biocompatible material employed by the arrayed stent graft consists of embodiments similar to those detailed in WO97/41164. In essence the biocompatible material consists of a polymer having both non-thrombogenic and anti-thrombogenic properties on the same polymer backbone or if required only non-thrombogenic or only anti-thrombogenic properties.

The polymers having non-thrombogenic properties can be prepared by copolymerising monomers of at least two classes from (a) monomers having sulphate groups, (b) monomers having sulphonate groups, (c) monomers having sulphamate groups, (d) monomers having polyoxyalkylene ether groups and (e) monomers having zwitterionic groups. The monomers can additionally be provided with anti-thrombogenic properties by including an additional co-monomer having pendant heparin (or hirudin, warfarin or hyaluronic acid) groups.

There have been many stent designs in the last decade designed to circumvent the problem of restenosis. In all these devices the problem has not been solved or reduced to a clinically acceptable level. Such devices include the Palmaz stent and the Gianturco stent, which have shown promise but still suffer from the problem of restenosis.

The inventors believe that the arrayed stent graft structure is collapsible, expandable and has mechanical integrity to maintain patency. Additionally the biocompatible material employed reduces inflammatory and thrombotic mechanisms whilst the stent array resists vessel recoil.

The invention is primarily concerned with those vessels, which are part of the vascular system of the human body. However, other applications for these devices are found in other body vessels and cavities. Typical examples of other uses include the biliary duct, and in transjugular intrahepatic portosystemic stent shunting (TIPSS).

The invention will be further described with reference to the various embodiments shown by way of example in the accompany drawings in which:
Fig. 1 is a side view of a stent element for use in an endoprosthesis according to the invention.
Fig. 2 is an end view of the stent element.
Fig. 3 is a side view of a forming jig used for winding Nitinol wire into a stent element.
Fig. 4 is a cross section view of the jig in Fig. 3.
Fig. 5 is an enlarged scrap view of the cross section shown in Fig. 4.
Fig. 6a is a view of the welded junction of the two ends of the wire in the stent element.
Fig. 6b is a view of the ends of the wire in the stent element joined by the use of a crimped-on or shrunk-on tube.
Fig. 6c is a view of the ends of the wire in the stent element joined by overlapping and binding by suture material.
Fig. 7 is a side view of an endoprosthesis with a number of stent elements sewn outside a barrier layer material. The stent elements are positioned radially and longitudinally such that the device has maximum flexibility.
Fig. 8 a cross section of the device in Fig. 7.
Fig. 9 is a side view of an endoprosthesis with a number of stent elements sewn inside a barrier layer material. The stent elements are positioned radially and longitudinally such that the device has maximum flexibility.
Fig. 10 is a cross section of the device in Fig. 9.
Fig. 11 is a side view of an endoprosthesis, similar to Fig. 7, where the stent elements are positioned radially and longitudinally so as to maximise length stability during the deployment of the device.
Fig. 12 is a side view of an endoprosthesis; similar to Fig. 9, where the stent elements are positioned radially and longitudinally so as to maximise length stability during the deployment of the device.
Fig. 13 illustrates another stent array where the number of individual stent elements are connected to each other by using an unbroken continuous wire in their construction. The continuation of the wire provides a spine or backbone for extra stability.
Fig. 14 shows an endoprosthesis in which the stent array of Fig. 13 sewn on to the outside of a barrier layer material. The connections between each individual stent crown are bound to give a spine or backbone to the device. The stent array is sewn to the barrier material to hold it open in use.
Fig. 15 is a side view of an endoprosthesis, similar to Fig. 7, in which the ends of the wire of individual stent elements are joined by binding together overlapping portions of the ends of wires forming individual stent elements.

The present invention is an endoprosthesis device suitable for the treatment of certain types of vascular disease, and disease of other body canals and vessels.

The device is a composite structure comprising one or more cylindrical spring stents or crowns and a barrier fabric or continuous tubular element. The device may be compacted into a small diameter in order to be delivered to the site of a lesion and then expanded to its normal diameter.

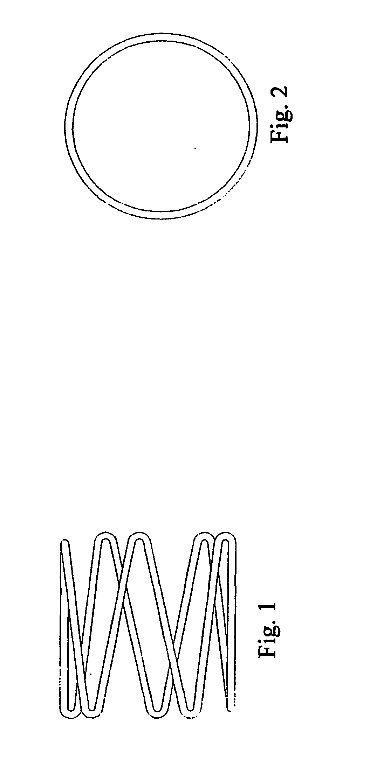

Fig. 1 shows a side view of one embodiment of the basic stent element. Fig. 2 shows the end view of the stent element. The stent element is made by winding a suitable wire into a number of ziz-zags to form a tubular element. The two ends of the wire in the element are connected to each other to form a continuous endless structure.

In a first embodiment of the stent element of the invention, the wire is made from a malleable metal, such as a wire of malleable stainless steel or other material with similar mechanical properties.

In a second embodiment of the stent component of the invention, the wire is made from a spring-tempered stainless steel or other materials with similar mechanical properties.

In both these embodiments, the wire may have a circular cross section or be in the form of a flat tape, or other cross section.

The method of manufacture of both the first and second embodiments of the stent element is that of the construction of standard wire components and is well known to those skilled in the arts of wire component construction and the manufacture of metallic mechanical springs.

In a third embodiment of the stent element, the wire is made from a shape memory alloy (SMA), e.g. Nitinol. Again, the wire used may have a number of different cross sections.

A wire of shape memory alloy may be formed into a shape, held in that shape by a jig or fixture, subjected to a heat treatment, and allowed to cool. This shape will then be "remembered" by the alloy.

If the so treated-formed shape is cooled below a critical temperature, it becomes malleable and may be deformed into a second shape. On heating above another critical temperature, the wire will return to its' "remembered" configuration.

Shape memory alloy may exist in one of two forms. Below a critical temperature it is martensitic or malleable, and above another critical temperature it is austenitic or spring like.

In its austenitic state, it may also display the property of "super elasticity" as described in the background to the invention above.

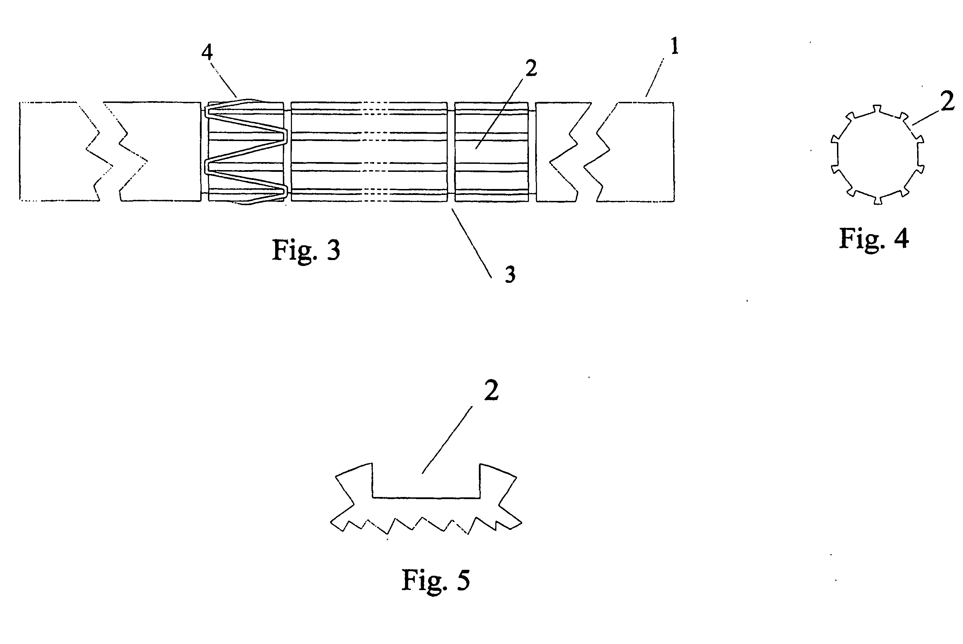

The stent element of shape memory alloy may be made by winding on to a cylindrical jig, similar to that shown in Fig. 3. This jig 1 has longitudinal slots 2 and circular grooves 3. The construction can be further visualised by reference to Fig. 4 and Fig. 5.

The wire 4 is wound into the slots and grooves to form the zigzag configuration. A number of stent elements may be wound side by side on the same jig.

After winding, the wire shape is held by sliding a close fitting tube over the wire and the jig.

The wound jig is then placed in a muffle furnace for heat treatment.

After the heat treatment, the jig and wire is cooled down to below the martensitic transformation temperature, and the wire unwound. After unwinding, the wire is placed in warm water when the remembered shape will return.

A suitable shape memory alloy, e.g. Nitinol would have a martensitic transform temperature of below 10°C and an austenitic transform temperature of about 30°C. A typical heat treatment would be 475°C for 15 minutes.

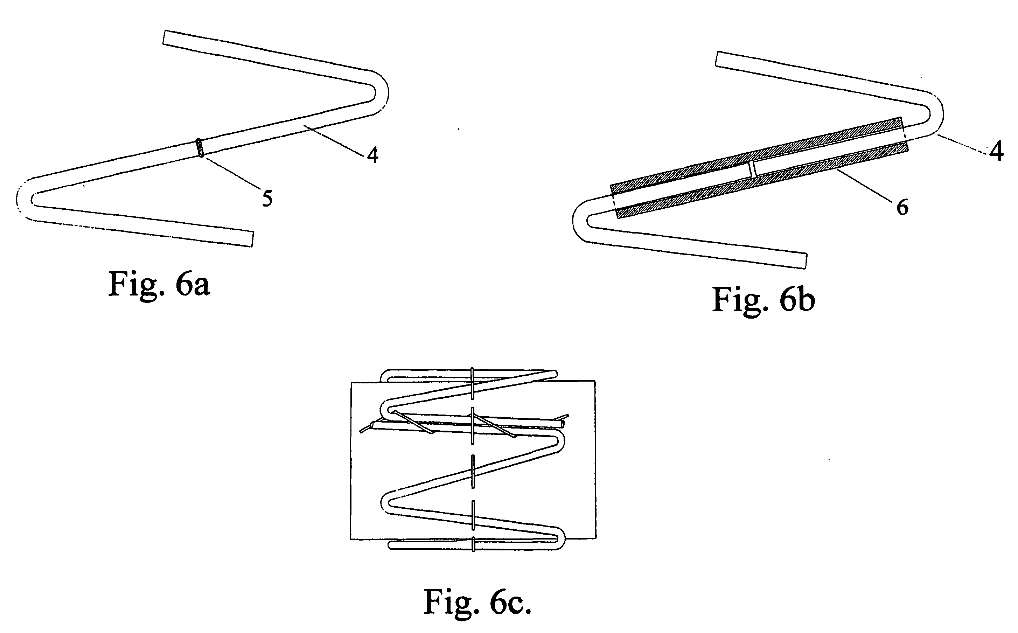

After the wire is formed the ends may be connected together by welding (Fig. 6a), or by the use of a suitable malleable tube crimped or glued in place (Fig. 6b).

Alternatively, as shown in Fig. 6c, the wire forming individual stent elements may be of such a length that the ends overlap one another. The overlapping ends can then be bound together, preferably by suture material as used to attach the stent elements to the continuous tubular element described below.

The crown elements used in the array can also be made by machining a metallic or polymeric tube. The machining may be by mechanical means, or may be cut by laser, or be formed by chemical etching.

The stent elements may be constructed in a number of different ways. One method is to wind a metallic or polymeric wire in a zigzag fashion as described above to form a crown element. This wire may have a circular cross section, or it may be a flat tape, or any of a number of different cross sections. The crown element may also be constructed from a metallic or polymeric tube by well known methods such as laser cutting and chemical etching.

The arrayed stent graft is made of a combination of one or more stent elements and a continuous tubular element.

The purpose of the continuous tubular element is to isolate the lumen of the artery from the blood and may be manufactured in a variety of ways.
1. Woven fabric. A fine tubular woven fabric
2. Knitted Fabric. Warp or weft Knitted tubular fabric
3. Tubular extrusion. A plastic tubular extrusion made from a variety of materials, which include: polyester, polyurethane, silicone elastomer, polypropylene, and polytetrafluoroethylene (PTFE)
4. Tubular film. A plastic tubular film made from a variety of materials, which include: polyester, polyurethane, silicone elastomer, polypropylene, and polytetrafluoroethylene (PTFE)

The endoprosthesis may be made in a number of different configurations, one with the stent elements on the inside of the tubular element, another with the stent elements on the outside of the tubular element and yet another where the stent elements are sandwiched between two tubular elements.

In the case of the tubular extrusion or tubular film, the tubular element may also encapsulate the stent array. Such encapsulation removes the need for suture material to connect the stent elements to the tubular element.

In addition, for each of these configurations there are a number of ways in which the stent elements may be arranged.

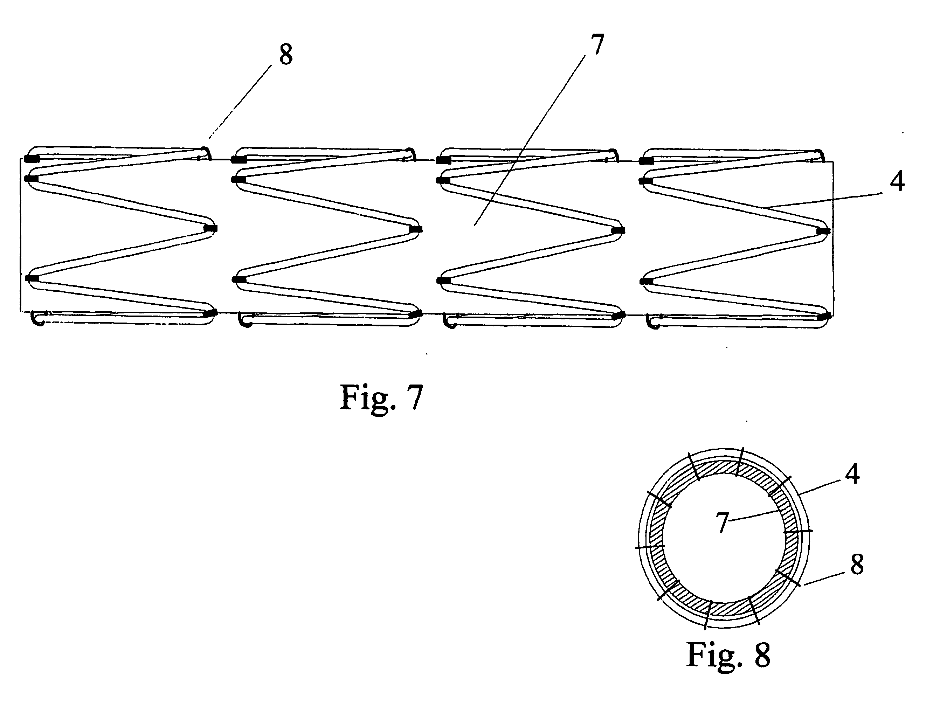

Fig. 7 shows one embodiment where the stent elements are on the outside of the continuous tubular element and the stent elements are arranged all in the same direction and spaced apart. This gives the maximum flexibility for the device.

The stent elements are placed outside the continuous tubular element and the apex of each stent element is attached to the continuous tubular element. The wire 4 is connected to the continuous tubular element 7 by attachments 8 at the apex of each zigzag. The attachments 8 may be filament sutures, welds, adhesives, clips or any other suitable device.

Fig 8 shows a cross section of this configuration.

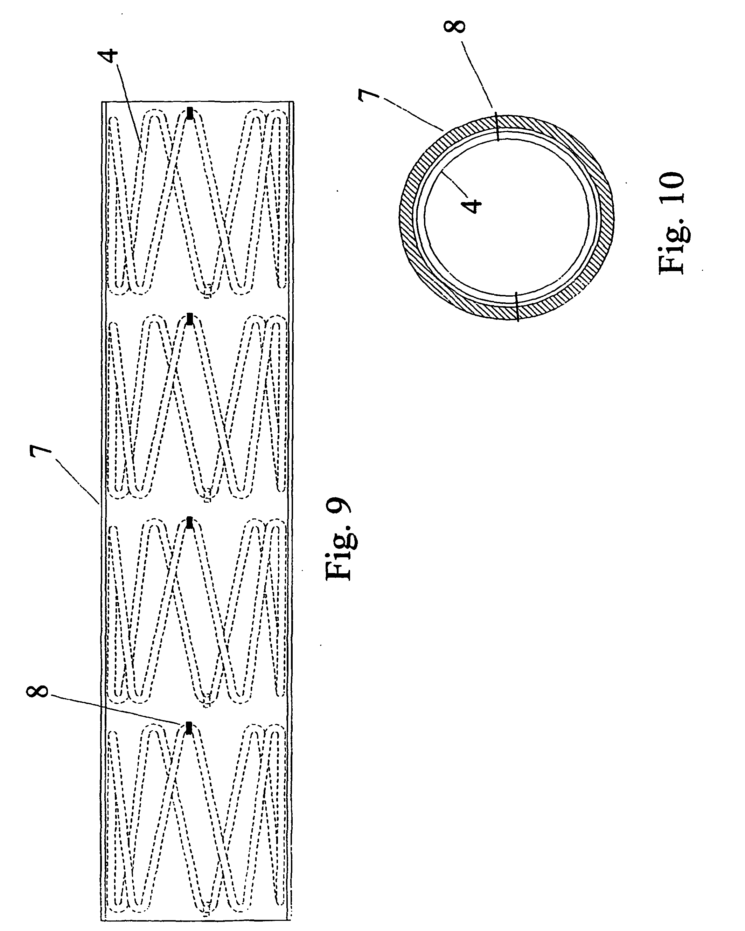

Fig. 9 shows another embodiment of the device where the stent elements are on the inside of the continuous tubular element. The wire 4 is connected to the continuous tubular element 7 by a number of sutures 8. Fewer sutures are needed when the continuous tubular element is on the outside since the continuous tubular element will be trapped between the stent elements and the lumen wall.

Fig. 10 shows a cross section of this arrangement.

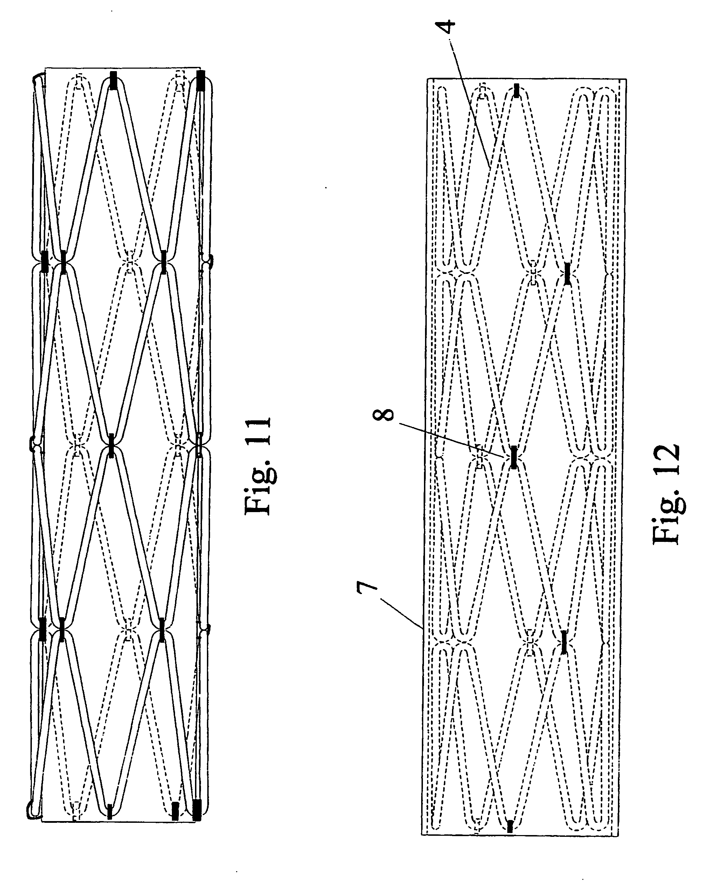

Fig. 11 shows an embodiment of the device where the stent elements are arranged with alternate stents reversed in direction. The stent elements are on the outside of the continuous tubular element, and therefore each apex must be attached to the continuous tubular element.

This arrangement puts the apexes of the zigzags from each stent element abutting each other. This configuration gives the maximum length stability when the device is pushed down a catheter during deployment.

Fig. 12 shows another embodiment where the continuous tubular element is on the outside of the stent elements.

Only a few of the apexes need to be sutured since the continuous tubular element will be trapped between the stent elements and the Luminal wall.

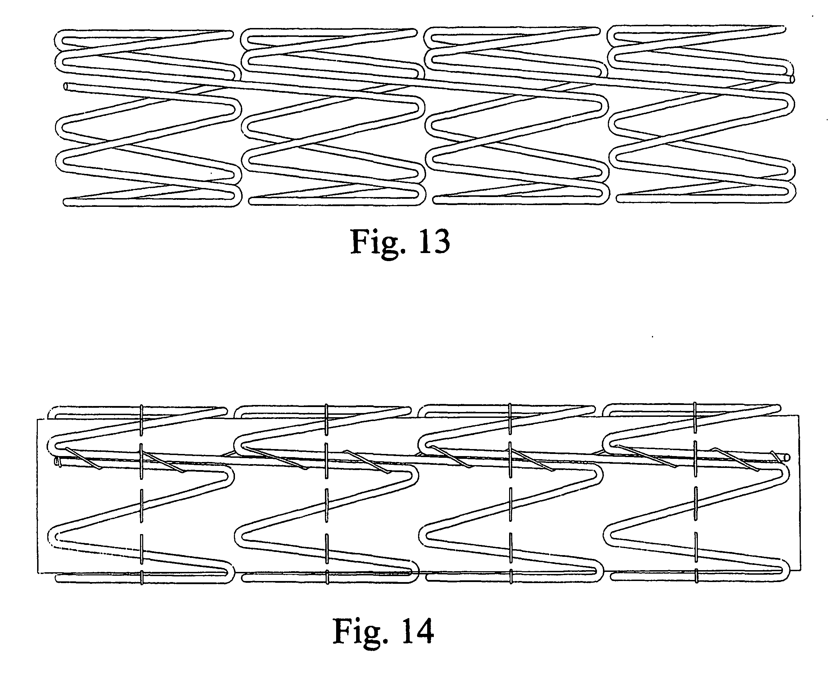

Fig. 13 shows another embodiment of the device. In this version the individual stent crowns are left connected to each other. The connecting wire acts as a spine, or backbone, to give greater stability. This length stability is especially useful during the deployment of the complete device.

Fig. 14 shows the connected stent array shown in Fig. 13, sutured to the outside of a continuous tubular element. The connecting sections of the wire are bound together by a suture material in order to form a continuous spine throughout the device. Since the stent array is on the outside of the continuous tubular element, the tubular material requires to be sewn to the stent array in order that the tubular material remains open after deployment. The spine adds stability to the complete array.

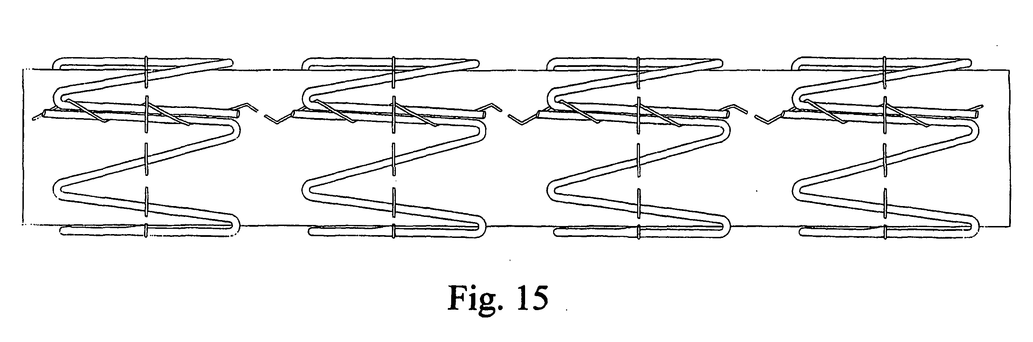

Fig. 15 shows a stent array sutured to an inner tubular element in a manner similar to Fig. 7, but with the arrayed stent elements constructed in the manner illustrated in Fig. 6c. Such stent elements may also be linked together in a similar manner to that illustrated in Figs. 9, 11 or 12.

Although not shown in Figs 14 and 15, the ends of the fabric tube needs to be bound by sutures, such binding to incorporate the outer apexes of the end stent elements. This is required to ensure that the ends of the tubular element are forced to remain open by the stent element.

The final process in the manufacture of the endoprosthesis is to apply the highly biocompatible layer on the external surface.

This biocompatible layer may be in the form of a thin coating or in the form of a hydrogel encapsulation.

The biocompatible layer consists of polymers having non-thrombogenic properties which can be prepared by copolymerising monomers of at least two classes from (a) monomers having sulphate groups, (b) monomers having sulphonate groups, (c) monomers having sulphamate groups, (d) monomers having polyoxyalkylene ether groups and (e) monomers having zwitterionic groups. The monomers can additionally be provided with anti-thrombogenic properties by including an additional co-monomer having pendant heparin (or hirudin, warfarin or hyaluronic acid) groups.

The biocompatible material employed by the arrayed stent graft comprises embodiments detailed in WO97/41164.

In one preferred method of application of the biocompatible material, detailed in WO97/41164, is initially to polymerise the desired monomers by conventional aqueous solution polymerisation using a water soluble initiator, such as potassium persulphate, after degassing the solution and under an inert gas such as nitrogen. Reaction temperature for polymerisation is at room or elevated temperature, provided that the heparin biological activity is not affected. The polymer may be purified by conventional means, such as precipitation, filtration, washing and dialysis.

The aforementioned biocompatible polymer is capable of being applied as a coating on the arrayed stent graft. In this regard modified polyethylene imine or other primary or secondary amino containing polymers form a stable attachment between the arrayed stent graft and the biocompatible polymer.

## Claims

1. A flexible endoprosthesis comprising an array of consecutive aligned cylindrical stent elements in the form of a plurality of sequentially connected radially stable outwardly biased cylindrical springs each constructed of wire wound in zigzag form and a one-piece tubular flexible material maintained in open tubular form by the array of stent elements, the array of stent elements and the tubular flexible material together being totally coated with or encapsulated in a biocompatible polymer layer comprising a polymer backbone having pendant groups, obtainable by polymerizing monomers having such groups, wherein said polymers are obtained by copolymerizing monomers of at least two different classes selected from:
a) monomers having sulphate groups
b) monomers having sulphonate groups
c) monomers having sulphamate groups
d) monomers having polyoxyalkylene ether groups, and
e) monomers having zwitterionic groups

2. An endoprosthesis as claimed in claim 1, **characterized in that** said polymers are obtained by copolymerizing monomers of at least two different classes selected from:
a) monomers having sulphate groups
b) monomers having sulphonate groups
c) monomers having sulphamate groups, and
d) monomers having polyoxyalkylene ether groups

3. An endoprosthesis as claimed in claim 1 or 2 **characterized in that** the polymers are obtained by copolymerizing monomers of two of the respective said different classes.

4. An endoprosthesis as claimed in any one of claims 1 to 3 **characterized in that** the polymer comprises an additional comonomer having pendant heparin, hirudin, warfarin or hyaluronic acid groups.

5. An endoprosthesis as claimed in any one of claims 1 to 4 **characterized in that** the biocompatible polymer layer coats or encapsulates the array of stent elements and the tubular flexible material in the form of a hydrogel.

6. An endoprosthesis as claimed in any one of claims 1 to 5 **characterized in that** the stent elements are permanently attached to the array of cylindrical stent elements.

7. An endoprosthesis as claimed in claim 6 **characterized in that** the tubular flexible material is attached by sewing, welding, an adhesive or mechanical clips.

8. An endoprosthesis as claimed in any one of claims 1 to 7 **characterized in that** the tubular flexible material is inside the stent elements.

9. An endoprosthesis as claimed in any one of claims 1 to 7 **characterized in that** the tubular flexible material is outside the stent elements.

10. An endoprosthesis as claimed in any one of claims 1 to 7 **characterized in that** the stent elements are sandwiched between two flexible tubes.

11. An endoprosthesis as claimed in any one of claims 1 to 7 **characterized in that** the stent elements are encapsulated by the tubular flexible material.

12. An endoprosthesis as claimed in any of claims 1 to 10 **characterized in that** the tubular flexible material is a textile fabric, a woven fabric, or a knitted fabric.

13. An endoprosthesis as claimed in claim 12 **characterized in that** the woven or knitted fabric is made from a polyester yarn.

14. An endoprosthesis as claimed in any of claims 1 to 12 **characterized in that** the tubular flexible material is a continuous tubular element or film.

15. An endoprosthesis as claimed in claim 14 **characterized in that** the continuous tubular element or film is formed from a synthetic polymer.

16. An endoprosthesis as claimed in claim 15 **characterized in that** the synthetic polymer is a polyester, or a polyurethane.

17. An endoprosthesis as claimed in claim 14 **characterized in that** the continuous tubular element or film is formed from an elastomer.

18. An endoprosthesis as claimed in claim 17 **characterized in that** the elastomer is silicone rubber or polytetrafluoroethylene.

19. An endoprosthesis as claimed in any of claims 1 to 18 **characterized in that** the stent elements are composed of a wire, wound, in zigzag form into a cylindrical shape.

20. An endoprosthesis as claimed in claim 19 **characterized in that** the wire has a circular cross section or is a flat tape.

21. An endoprosthesis as claimed in any of claims 1 to 18 **characterized in that** the stent elements are constructed from a metallic or polymeric tube by laser cutting or chemical etching.

22. An endoprosthesis as claimed in claim 21 **characterized in that** the wire of each stent element has both ends connected to each other so as to have a continuous form.

23. An endoprosthesis as claimed in claim 19 **characterized in that** the ends of the wire of each stent element are joined by overlapping and binding with suture material.

24. An endoprosthesis as claimed in any of claims 1 to 19
**characterized in that** the individual stent elements are made from a continuous length of wire so that they remain connected to each other.

25. An endoprosthesis as claimed in any of claims 17 to 24 **characterized in that** the stent elements are made from spring-tempered metal.

26. An endoprosthesis as claimed in claim 25 **characterized in that** the spring-tempered metal is stainless steel.

27. An endoprosthesis as claimed in any of claims 17 to 24 **characterized in that** the stent elements are made from a shape memory alloy.

28. An endoprosthesis as claimed in claim 27 **characterized in that** the shape memory alloy wire is martensitic at temperatures lower than 37°C.

29. An endoprosthesis as claimed in claim 27 **characterized in that** the shape memory alloy wire is austenitic at or above a temperature of 37°C.

30. An endoprosthesis as claimed in claim 27 **characterized in that** the shape memory alloy wire is in superelastic form at or above a temperature of 37°C.

31. An endoprosthesis as claimed in any of claims 17 to 24 **characterized in that** the stent elements are made from a malleable material.

32. An endoprosthesis as claimed in claim 31 **characterized in that** the malleable material is malleable stainless steel.

33. An endoprosthesis as claimed in any of claims 1 to 32 **characterized in that** the individual stent elements are arranged so as not to touch each other or to interfere with each other so as to give maximum flexibility to the complete device during delivery and subsequent operation.

34. An endoprosthesis as claimed in any of claims 1 to 32 **characterized in that** the individual stent elements are arranged so as to be alternately of opposite phase with the apexes of the zigs in one stent element in contact with the next, so as to give maximum stability to the device during delivery.

35. An endoprosthesis as claimed in claim 1 to 32 **characterized in that** the connections between individual stent elements are bound together to form a longitudinal spine in the complete device.

## Patentansprüche

1. Eine flexible Endoprothese, enthaltend eine regelmäßige Anordnung von aufeinander folgenden zueinander ausgerichteten zylindrischen Stent-Elementen in der Form von einer Vielzahl von aufeinander folgend aneinander angeschlossenen, radial stabilen, nach außen vorgespannten Zylinderfedem, von denen jede aus Draht hergestellt ist, der in Zick-Zack-Form gewickelt ist, und ein einstückiges, rohrförmiges, flexibles Material, das in durch die Anordnung der Stent-Elemente offener Rohrform gehalten wird, wobei die Anordnung von Stent-Elementen und das rohrförmige, flexible Material zusammen vollständig beschichtet sind mit oder eingekapselt sind in eine biokompatible Polymerschicht, enthaltend ein Polymerrückgrat mit anhängenden Gruppen, die erhältlich sind durch Polymerisieren von Monomeren, die solche Gruppen haben, wobei diese Polymere erhalten worden sind durch Copolymerisieren von Monomeren aus mindestens zwei verschiedenen Klassen, die ausgewählt sind aus:
a) Monomeren mit Sulfatgruppen
b) Monomeren mit Sulfonatgruppen
c) Monomeren mit Sulfamatgruppen
d) Monomeren mit Polyoxyalkylenethergruppen, und
e) Monomeren mit zwitterionischen Gruppen

2. Eine Endoprothese nach Anspruch 1, **dadurch gekennzeichnet, daß** diese Polymere erhalten worden sind durch Copolymerisation von Monomeren aus mindestens zwei unterschiedlichen Klassen, die ausgewählt sind aus:
a) Monomeren mit Sulfatgruppen
b) Monomeren mit Sulfonatgruppen
c) Monomeren, die Sulfonatgruppen haben, und
d) Monomeren, die Polyoxyalkylenethergruppen haben.

3. Eine Endoprothese nach Anspruch 1 oder 2, **dadurch gekennzeichnet, daß** die Polymere erhalten worden sind durch Copolymerisation von Monomeren aus zwei der jeweiligen dieser unterschiedlichen Klassen.

4. Eine Endoprothese nach einem der Ansprüche 1 bis 3, **dadurch gekennzeichnet, daß** das Polymer ein zusätzliches Comonomer enthält, das anhängende Heparin-, Hirudin-, Warfarin- oder Hyaluronsäuregruppen hat.

5. Eine Endoprothese nach einem der Ansprüche 1 bis 4, **dadurch gekennzeichnet, daß** die biokompatible Polymerschicht die Anordnung von Stentelementen und das rohrförmige, flexible Material in Form eines Hydrogels beschichtet oder einkapselt.

6. Eine Endoprothese nach einem der Ansprüche 1 bis 5, **dadurch gekennzeichnet, daß** die Stent-Elemente an dieser Anordnung von zylindrischen Stent-Elementen permanent angebracht sind.

7. Eine Endoprothese nach Anspruch 6, **dadurch gekennzeichnet, daß** das rohrförmige, flexible Material durch Vernähen, Schweißen, einen Klebstoff oder mittels mechanischer Clips angebracht ist.

8. Eine Endoprothese nach einem der Ansprüche 1 bis 7, **dadurch gekennzeichnet, daß** das rohrförmige, flexible Material sich innerhalb der Stent-Elemente befindet.

9. Eine Endoprothese nach einem der Ansprüche 1 bis 7, **dadurch gekennzeichnet, daß** das rohrförmige, flexible Material sich außerhalb der Stent-Elemente befindet.

10. Eine Endoprothese nach einem der Ansprüche 1 bis 7, **dadurch gekennzeichnet, daß** die Stent-Elemente sandwichartig zwischen zwei flexiblen Rohren angeordnet sind.

11. Eine Endoprothese nach einem der Ansprüche 1 bis 7, **dadurch gekennzeichnet, daß** die Stent-Elemente von dem rohrförmigen, flexiblen Material eingekapselt sind.

12. Eine Endoprothese nach einem der Ansprüche 1 bis 10, **dadurch gekennzeichnet, daß** das rohrförmige, flexible Material ein Textilstoff, ein Webstoff oder ein Strickstoff ist.

13. Eine Endoprothese nach Anspruch 12, **dadurch gekennzeichnet, daß** der Web- oder Strickstoff aus einem Polyestergarn hergestellt ist.

14. Eine Endoprothese nach einem der Ansprüche 1 bis 12 , **dadurch gekennzeichnet, daß** das rohrförmige, flexible Material ein fortlaufendes Rohrelement oder ein Film ist.

15. Eine Endoprothese nach Anspruch 14, **dadurch gekennzeichnet, daß** das fortlaufende Rohrelement oder der Film aus einem synthetischen Polymer hergestellt ist.

16. Eine Endoprothese nach Anspruch 15, **dadurch gekennzeichnet, daß** das synthetische Polymer ein Polyester oder ein Polyurethan ist.

17. Eine Endoprothese nach Anspruch 14, **dadurch gekennzeichnet, daß** das fortlaufende, rohrförmige Element oder der Film aus einem Elastomer hergestellt ist.

18. Eine Endoprothese nach Anspruch 17, **dadurch gekennzeichnet, daß** das Elastomer Silicongummi oder Polytetrafluorethylen ist.

19. Eine Endoprothese nach einem der Ansprüche 1 bis 18, **dadurch gekennzeichnet, daß** die Stent-Elemente zusammengesetzt sind aus einem Draht, der in Zick-Zack-Form zu einer zylindrischen Form gewickelt ist.

20. Eine Endoprothese nach Anspruch 19, **dadurch gekennzeichnet, daß** der Draht einen kreisförmigen Querschnitt hat oder ein flaches Band ist.

21. Eine Endoprothese nach einem der Ansprüche 1 bis 18, **dadurch gekennzeichnet, daß** die Stent-Elemente durch Laserschneiden oder chemischen Ätzen aus einem Metall- oder Polymerrohr hergestellt sind.

22. Eine Endoprothese nach Anspruch 21, **dadurch gekennzeichnet, daß** der Draht jedes Stent-Elementes beide Enden aneinander angeschlossen hat, so daß er eine fortlaufende Form hat.

23. Eine Endoprothese nach Anspruch 19, **dadurch gekennzeichnet, daß** die Drahtenden jedes Stent-Elementes durch Überlappen und Verbinden mit Verbindungsmaterial miteinander verbunden sind.

24. Eine Endoprothese nach einem der Ansprüche 1 bis 19, **dadurch gekennzeichnet, daß** die einzelnen Stent-Elemente aus einer fortlaufenden Drahtlänge hergestellt sind, so daß sie aneinander angeschlossen bleiben.

25. Eine Endoprothese nach einem der Ansprüche 17 bis 24, **dadurch gekennzeichnet, daß** die Stent-Elemente aus einem Feder-Vergütungsmetall hergestellt sind.

26. Eine Endoprothese nach Anspruch 25, **dadurch gekennzeichnet, daß** das Feder-Vergütungsmetall ein nicht rostender Stahl ist.

27. Eine Endoprothese nach einem der Ansprüche 17 bis 24, **dadurch gekennzeichnet, daß** die Stent-Elemente hergestellt sind aus einer Formgedächtnislegierung.

28. Eine Endoprothese nach Anspruch 27, **dadurch gekennzeichnet, daß** der Formgedächtnislegierungsdraht bei einer Temperatur unterhalb von 37 °C martensitisch ist.

29. Eine Endoprothese nach Anspruch 27, **dadurch gekennzeichnet, daß** der Formgedächtnislegierungsdraht bei einer Temperatur von 37 °C oder darüber austenitisch ist.

30. Eine Endoprothese nach Anspruch 27, **dadurch gekennzeichnet, daß** der Formgedächtnislegierungsdraht bei einer Temperatur von 37 °C oder darüber in superelastischer Form vorliegt.

31. Eine Endoprothese nach einem der Ansprüche 17 bis 24, **dadurch gekennzeichnet, daß** die Stent-Elemente aus einem verformbaren Material hergestellt sind.

32. Eine Endoprothese nach Anspruch 31, **dadurch gekennzeichnet, daß** das verformbare Material ein verformbarer rostfreier Stahl ist.

33. Eine Endoprothese nach einem der Ansprüche 1 bis 32, **dadurch gekennzeichnet, daß** die einzelnen Stent-Elemente so angeordnet sind, daß sie sich nicht miteinander berühren oder miteinander interferieren, um dem gesamten Gerät während des Einsetzens und eines anschließenden Eingriffs eine maximale Flexibilität zu verleihen.

34. Eine Endoprothese nach einem der Ansprüche 1 bis 32, **dadurch gekennzeichnet, daß** die einzelnen Stent-Elemente so angeordnet sind, daß sie abwechselnd von entgegen gesetzten Phasen mit den Scheitelbereichen der Zick-Zack-Kehren in einem Stent-Element in Kontakt sind mit dem nächsten, so daß dem Gerät während des Einsetzens eine maximale Stabilität verliehen ist.

35. Eine Endoprothese nach einem der Ansprüche 1 bis 32, **dadurch gekennzeichnet, daß** die Anschlüsse zwischen einzelnen Stent-Elementen miteinander verbunden sind, um in dem Gesamtgerät ein Längsrückgrat zu bilden.

## Revendications

1. Endoprothèse flexible comprenant une série d'éléments formant stents cylindriques alignés et consécutifs se présentant sous la forme d'une pluralité de ressorts cylindriques sollicités vers l'extérieur, reliés de manière séquentielle, et stables de manière radiale, chacun se composant de fil enroulé en zigzags et un matériau flexible tubulaire en une pièce maintenu dans une forme tubulaire ouverte par la série d'éléments formant stents, la série d'éléments formant stents et le matériau flexible tubulaire étant conjointement complètement enduits ou enveloppés d'une couche polymérique biocompatible comprenant une chaîne polymérique comportant des groupes pendants, pouvant être obtenue en polymérisant des monomères comportant ces groupes, dans laquelle lesdits polymères sont obtenus en copolymérisant des monomères d'au moins deux classes différentes choisies parmi :
a) des monomères comportant des groupes sulfate ;
b) des monomères comportant des groupes sulfonate ;
c) des monomères comportant des groupes sulfamate ;
d) des monomères comportant des groupes polyoxyalkylène éther, et
e) des monomères comportant des groupes zwitterioniques.

2. Endoprothèse selon la revendication 1, **caractérisée en ce que** lesdits polymères sont obtenus en copolymérisant des monomères d'au moins deux classes différentes choisies parmi :
a) des monomères comportant des groupes sulfate ;
b) des monomères comportant des groupes sulfonate ;
c) des monomères comportant des groupes sulfamate ; et
d) des monomères comportant des groupes polyoxyalkylène éther.

3. Endoprothèse selon la revendication 1 ou 2, **caractérisée en ce que** les polymères sont obtenus en copolymérisant des monomères de deux desdites classes différentes respectives.

4. Endoprothèse selon l'une quelconque des revendications 1 à 3, **caractérisée en ce que** le polymère comprend un comonomère supplémentaire comportant des groupes pendants héparine, hirudine, warfarine ou acide hyaluronique.

5. Endoprothèse selon l'une quelconque des revendications 1 à 4, **caractérisée en ce que** la couche polymérique biocompatible revêt ou enveloppe la série d'éléments formant stents et le matériau flexible tubulaire sous la forme d'un hydrogel.

6. Endoprothèse selon l'une quelconque des revendications 1 à 5, **caractérisée en ce que** les éléments formant stents sont fixés de manière permanente à la série d'éléments formant stents cylindriques.

7. Endoprothèse selon la revendication 6, **caractérisée en ce que** le matériau flexible tubulaire est fixé par couture, soudure, par un adhésif ou des pinces mécaniques.

8. Endoprothèse selon l'une quelconque des revendications 1 à 7, **caractérisée en ce que** le matériau flexible tubulaire se trouve à l'intérieur des éléments formant stents.

9. Endoprothèse selon l'une quelconque des revendications 1 à 7, **caractérisée en ce que** le matériau flexible tubulaire se trouve à l'extérieur des éléments formant stents.

10. Endoprothèse selon l'une quelconque des revendications 1 à 7, **caractérisée en ce que** les éléments formant stents sont pris en sandwich entre deux tubes flexibles.

11. Endoprothèse selon l'une quelconque des revendications 1 à 7, **caractérisée en ce que** les éléments formant stents sont enveloppés par le matériau flexible tubulaire.

12. Endoprothèse selon l'une quelconque des revendications 1 à 10, **caractérisée en ce que** le matériau flexible tubulaire est un tissu, un textile tissé, ou un tissu à mailles.

13. Endoprothèse selon la revendication 12, **caractérisée en ce que** le textile tissé ou le tissu à mailles se compose d'un fil de polyester.

14. Endoprothèse selon l'une quelconque des revendications 1 à 12, **caractérisée en ce que** le matériau flexible tubulaire est un élément ou un film tubulaire continu.

15. Endoprothèse selon la revendication 14, **caractérisée en ce que** l'élément ou le film tubulaire continu est formé à partir d'un polymère synthétique.

16. Endoprothèse selon la revendication 15, **caractérisée en ce que** le polymère synthétique est un polyester, ou un polyuréthanne.

17. Endoprothèse selon la revendication 14, **caractérisée en ce que** l'élément ou le film tubulaire continu est formé à partir d'un élastomère.

18. Endoprothèse selon la revendication 17, **caractérisée en ce que** l'élastomère est du caoutchouc de silicone ou du polytétrafluoréthylène.

19. Endoprothèse selon l'une quelconque des revendications 1 à 18, **caractérisée en ce que** les éléments formant stents se composent d'un fil enroulé en zigzags pour adopter une forme cylindrique.

20. Endoprothèse selon la revendication 19, **caractérisée en ce que** le fil présente une section transversale circulaire ou est un ruban plat.

21. Endoprothèse selon l'une quelconque des revendications 1 à 18, **caractérisée en ce que** les éléments formant stents sont fabriqués à partir d'un tube métallique ou polymérique par découpe par laser ou par décapage chimique.

22. Endoprothèse selon la revendication 21, **caractérisée en ce que** les deux extrémités du fil de chaque élément formant stent sont reliées l'une à l'autre de manière à présenter une forme continue.

23. Endoprothèse selon la revendication 19, **caractérisée en ce que** les extrémités du fil de chaque élément formant stent sont jointes par chevauchement et liaison à l'aide d'un matériau de suture.

24. Endoprothèse selon l'une quelconque des revendications 1 à 19, **caractérisée en ce que** les éléments formant stents individuels se composent d'une longueur continue de fil de telle sorte qu'ils restent reliés les uns aux autres.

25. Endoprothèse selon l'une quelconque des revendications 17 à 24, **caractérisée en ce que** les éléments formant stents sont réalisés dans du métal revenu à l'état ressort.

26. Endoprothèse selon la revendication 25, **caractérisée en ce que** le métal revenu à l'état ressort est de l'acier inoxydable.

27. Endoprothèse selon l'une quelconque des revendications 17 à 24, **caractérisée en ce que** les éléments formant stents sont réalisés dans un alliage à mémoire de forme.

28. Endoprothèse selon la revendication 27, **caractérisée en ce que** le fil en alliage à mémoire de forme est martensitique à des températures inférieures à 37°C.

29. Endoprothèse selon la revendication 27, **caractérisée en ce que** le fil en alliage à mémoire de forme est austénitique à une température supérieure ou égale à 37°C.

30. Endoprothèse selon la revendication 27, **caractérisée en ce que** le fil en alliage à mémoire de forme se présente sous une forme superélastique à une température supérieure ou égale à 37°C.

31. Endoprothèse selon l'une quelconque des revendications 17 à 24, **caractérisée en ce que** les éléments formant stents sont réalisés dans un matériau malléable.

32. Endoprothèse selon la revendication 31, **caractérisée en ce que** le matériau malléable est de l'acier inoxydable malléable.

33. Endoprothèse selon l'une quelconque des revendications 1 à 32, **caractérisée en ce que** les éléments formant stents individuels sont agencés de manière à ne pas se toucher les uns les autres ou à ne pas interférer les uns avec les autres afin de conférer une flexibilité maximale à tout le dispositif pendant la distribution et l'opération suivante.

34. Endoprothèse selon l'une quelconque des revendications 1 à 32, **caractérisée en ce que** les éléments formant stents individuels sont agencés de manière à être, de manière alternative, de phase opposée, les sommets des zigzags dans un élément formant stent étant en contact avec les suivants, de manière à conférer une stabilité maximale au dispositif pendant la distribution.

35. Endoprothèse selon l'une quelconque des revendications 1 à 32, **caractérisée en ce que** les connexions entre les éléments formant stents individuels sont liées les unes aux autres de manière à former une chaîne longitudinale dans tout le dispositif.
